# EUROPÄISCHE PATENTSCHRIFT

(11) **EP 3 672 657 B1**
(45) Veröffentlichungstag und Bekanntmachung des Hinweises auf die Patenterteilung: **13.10.2021**
(21) Anmeldenummer: 18768767.8
(22) Anmeldetag: 21.08.2018
(51) Int. Cl.: A61M 1/06

(54) **BABYMUNDSIMULATOR**
BABY MOUTH SIMULATOR
SIMULATEUR DE BOUCHE DE BÉBÉ

(30) Priorität: 21.08.2017 DE 102017119034
(43) Veröffentlichungstag der Anmeldung: 01.07.2020
(73) Patentinhaber: MAM Baby AG, 8832 Wollerau (CH)
(72) Erfinder: Kurth, Elisabeth, 01445 Radebeul (DE)
(74) Vertreter: SONN Patentanwälte OG
(86) Internationale Anmeldenummer: PCT/EP2018/072494
(87) Internationale Veröffentlichungsnummer: WO 2019/038255

(56) Entgegenhaltungen:
- WO-A1-2016/014469
- DE-A1-102014 103 567

## Beschreibung

Die Erfindung betrifft einen Babymundsimulator für die weibliche Brust mit einem Aufnahmeraum für die laktierte Milch. Weiter weist dieser eine Melkmanschette mit einem flexiblen Teil auf, mit dem der Babymundsimulator auf die weibliche Brust dicht aufsetzbar ist. Diese Melkmanschette ist derart ausgebildet, dass ein manueller eine Laktation bewirkender Melkdruck auf die Mamille und die angrenzenden Bereiche ausführbar ist.

Aus der DE 10 2014 103 567 A1 ist ein Milchentnahmemodul für die weibliche Brust bekannt. Diese weist einen Auffangbehälter für die laktierte Milch auf. Darin werden verschiedene Methoden des Einbringens einer Melkbewegung oder eines Melkdrucks beschrieben. Diese bestehen in einer "Daumen-oben-Finger-unten-Methode" oder einer "zwei-bis-zehn-Finger-Methode", nachfolgend als "Breitfinger-Methode" bezeichnet und einer "Fünf-Finger-Methode", nachfolgend als "Spitzfinger-Methode" bezeichnet. Mit beiden Methoden wird auf physiologische Art eine rasche und dauerhafte Milchejektion erreicht. Das Verfahren zur Erzeugung einer Laktation sowie Funktion und Wirkung des Milchentnahmemoduls sind in dieser Druckschrift ausführlich beschrieben.

Dieses bekannte Milchentnahmemodul ist als eine, eine Öffnung für die laktierte Milch aufweisende Melkmanschette ausgebildet. Die Melkmanschette weist einen flexiblen Teil auf, mit dem sie auf die weibliche Brust dicht aufsetzbar ist. Dabei ist sie derart ausgebildet, dass ein manueller eine Laktation bewirkender Melkdruck auf die Mamille und die angrenzenden Bereiche ausführbar ist, wobei an der Öffnung ein Ventil angeordnet ist. Es hat sich nun in der Praxis gezeigt, dass bei der Erzeugung einer Laktation mit dem Milchentnahmemodul ein Teil der Milch nicht in das Aufnahmegefäß gelangt und der Unterdruck öfter verlorengeht.

Aus der WO 2016/014469 A1 ist weiters ein elektrisches Brustpumpensystem bekannt, bei welchem ein Brustadapter vorgesehen ist, der eine flexible Saugzone aufweist, mit welcher eine dichte Anlage zur Mutterbrust erzielt wird, wenn ein Saugdruck angelegt wird. Zusätzlich zu dem konstant angelegten Unterdruck für eine dichte Anlage kann ein zweiter größerer Saugdruck in einem dem Nippel umgebenden Raum angelegt werden.

Die Aufgabe der Erfindung besteht daher darin, die Effektivität des Verfahrens einer manuellen Erzeugung einer Laktation der weiblichen Brust zu verbessern. Die Effektivität wird dabei durch eine Vergrößerung der aufgefangenen Milchmenge in kürzerer Zeit, insbesondere sowohl durch eine Verbesserung des manuellen Einwirkens, längeres Halten des Unterdrucks als auch durch eine Vermeidung von Verlusten bestimmt.

Diese Aufgabe wird durch einen Babymundsimulator mit den Kennzeichen des Anspruches 1 gelöst. Die Ansprüche 2 bis 15 geben besondere Ausgestaltungen wieder.

Ein erfindungsgemäßer Babymundsimulator der eingangs genannten Art sieht vor, dass die Melkmanschette als Doppelmanschette ausgebildet ist, wobei eine den Aufnahmeraum beim bestimmungsgemäßen Einsatz gegenüber der Areola abdichtenden inneren Dichtmanschette und eine beim bestimmungsgemäßen Einsatz den Babymundsimulator auf der Brust mechanisch fixierende Fixierungsmanschette vorgesehen ist und dass der Aufnahmeraum als Vakuumraum ausgebildet ist.

Ein solcher Babymundsimulator ist sowohl für die Anwendung der Spitz- als auch der Breitfingermethode geeignet. Bei der Spitzfingermethode werden die Fingerkuppen einer Hand axial zur Mamille gehalten, um damit durch eine den Aufnahmeraum umgebenden Wandung hindurch einen Melkdruck auf die Mamille und die angrenzenden Bereiche auszuüben. Bei der Breitfingermethode werden die Finger radial oder tangential zur Mamille gehalten. Der Aufnahmeraum kann bei beiden Methoden von außen mit den Fingern verformt werden, wobei das Aufbringen eines mechanischen Drucks der Stimulation durch den Mundraum eines Säuglings entspricht. Die innere Dichtmanschette sitzt dabei - den Aufnahmeraum gegenüber der Brust abdichtend - auf der Brust insbesondere im Bereich der Areola auf. Durch Fingerdruck wird der Aufnahmeraum zusammengedrückt, die Luft entweicht zum Teil aus dem Aufnahmeraum und ein Unterdruck oder ein leichtes Vakuum entsteht. Dies entspricht dem Saugen eines Säuglings. Somit können durch den erfindungsgemäßen Babymundsimulator sämtliche wichtigen Faktoren zur Bewirkung einer Laktation auf natürliche Art und Weise und mit einer eigenen gefühlsmäßigen Rückkopplung simuliert werden.

Die äußere Fixierungsmanschette entfaltet keine Dichtwirkung. Sie dient vielmehr in erster Linie dem Aufsetzen des Babymundsimulators auf die Brust und dabei der mechanischen Fixierung. So ist es beispielsweise möglich, mit einer Hand die Fixierungsmanschette auf die Brust zu drücken und mit der anderen Hand die Melkbewegung auszuführen. Aber auch ein Melken mit beiden Händen ist möglich, wobei dabei die Finger oder die Handballen auf der Fixierungsmanschette aufliegen und der Babymundsimulator dadurch mechanisch fixiert wird.

Da die Fixierungsmanschette keine Dichtfunktion aufweist sondern die Fixierung des Babymundsimulators auf der Brust zur Aufgabe hat, kann diese verschiedene Formen aufweisen, die dieser Aufgabe gerecht werden. In einer Ausführungsform ist jedoch vorgesehen, dass die
Fixierungsmanschette größer als die Dichtmanschette (15) ist und die Dichtmanschette umgibt.

In einer Ausgestaltung ist vorgesehen, dass der Aufnahmeraum eine Öffnung aufweist, die mit einem Ventil, insbesondere einem Rückschlagventil, welches insbesondere als Lippenventil ausgebildet ist, versehen ist. Damit kann die Luft in dem Aufnahmeraum durch das Ventil entweichen, wenn der Aufnahmeraum zusammengedrückt wird. Entlasten die Finger den Aufnahmeraum wieder, hat dieser infolge des flexiblen Materials das Bestreben, in seine alte Form zurückzukehren, wodurch das Vakuum entsteht, wenn das Ventil geschlossen wird, was besonders einfach durch eine Gestaltung als Rückschlagventil geschehen kann. Eine Realisierung als Lippenventil stellt hierbei eine bei dem weichelastischen Material, welches zum Einsatz kommen wird, eine sehr leicht herzustellende Realisierung dar.

In einer weiteren Ausgestaltung ist ein den Aufnahmeraum umschließender Verstärkungsring vorgesehen. Dieser Verstärkungsring dient im Wesentlichen dem mechanischen Schutz der Mamille, da dieser den Raum um die Mamille offenhält.

Der Babymundsimulator kann auch zweiteilig ausgebildet sein. Damit können einerseits fertigungstechnische Erleichterungen erreicht werden. Andererseits wird es dadurch leichter möglich, verschiedene Materialien zum Einsatz zu bringen.

Der Babymundsimulator kann derart ausgebildet sein, dass der flexible Teil aus einem weichelastischen Material besteht. Ein solches Material erlaubt in besonders zweckmäßiger Weise eine Verformung und ein Abdichten des Aufnahmeraumes.

Eine weitere Ausgestaltung der Erfindung besteht darin, dass die Teile des Babymundsimulators aus verschiedenen Materialen bestehen. So wird es möglich, weichelastische Materialien mit steiferen Materialen zu kombinieren.

In einer Ausgestaltung ist ein in den Aufnahmeraum einsteckbarer Mamillenschutzkörper vorgesehen. Dieser Mamillenschutzkörper bietet - ähnlich dem bereits oben beschriebenen Verstärkungsring - einen Schutz der Mamille vor einer zu kräftigen Melkbewegung. Der Mamillenschutzring kann zusätzlich zu dem Verstärkungsring vorgesehen werden oder alternativ dazu. Insbesondere ist der Mamillenschutzkörper einsetzbar bei einem Babymundsimulator, der für die Spitzfingermethode mit äußeren Ausstülpungen versehen ist, wie dies weiter unter beschrieben ist, da bei dieser Lösung das Anbringen eines Verstärkungsringes nicht sinnvoll ist.

In einer Ausgestaltung der Erfindung kann der Mamillenschutzkörper auch für mehrere Funktionen genutzt werden. Dabei wird dieser so ausgebildet, dass er in den Vakuumraum dicht einbringbar ist und die Dichtmanschette mit dem Mamillenschutzkörper verbunden ist.

Der Babymundsimulator kann auch dadurch ausgestaltet sein, dass der Aufnahmeraum an seiner Außenseite mit Ausstülpungen versehen ist, in denen Teile der Finger einer Hand aufnehmbar sind. Diese Ausstülpungen bilden eine Art Handschuh für Finger. Diese sind derart ausgebildet, dass sich die weibliche Brust/Areola mit den Fingern einer Hand oder beider Hände durch die Wandung des Aufnahmeraumes hindurch leicht umschließen lässt und ein manueller, eine Laktation bewirkender Melkdruck auf die Mamille und die angrenzenden Bereiche mit den Fingern ausgeführt werden kann.

Dabei sollten die Ausstülpungen derart ausgebildet sein, dass zumindest die Fingerkuppen der Hand darin aufnehmbar sind und die Ausstülpungen den Aufnahmeraum umgeben.

Zur Realisierung eines Babymundsimulators, der sowohl für die Breitfingermethode als auch für die Spitzfinger-Methode genutzt werden kann, ist es vorgesehen, dass im Verhältnis zu den Ausstülpungen steifere Offenhalter vorgesehen sind, die in die Ausstülpungen anstelle der Fingerkuppen einsteckbar sind. Ohne diese Offenhalter ist dann der Babymundsimulator für die Spitzfingermethode einsetzbar. Werden die Offenhalter in die Ausstülpungen eingefügt, ist diese Anordnung auch für die Breitfingermethode nutzbar.

In einer Ausgestaltung ist vorgesehen, dass mit dem Aufnahmeraum ein Auffanggefäß verbindbar ist. In dieses Auffanggefäß kann dann die laktierte Milch abfließen. Zweckmäßigerweise ist in dem Auffanggefäß der gleiche Druck vorgesehen, wie in dem Aufnahmeraum, so dass die Milch ohne zwischengeschaltetes Ventil oder Pumpe abfließen kann.

Für den Fall, dass keine Druckgleichheit zwischen Aufnahmeraum und Auffanggefäß eingestellt werden kann, ist vorgesehen, dass das Ventil oder ein zweites Ventil zwischen dem Aufnahmeraum und dem Auffanggefäß angeordnet ist.

Vorteilhaft kann es sein, wenn das Auffanggefäß mit einer Armmanschette zur Befestigung insbesondere am Handgelenk oder am Unterarm versehen ist. Somit kann das Auffanggefäß bequem gehalten werden.

Es ist auch möglich, dass eine einen mechanischen Vibrator aufnehmbare Aufnahmetasche mit dem Babymundsimulator verbunden ist. Mittels eines solchen Vibrators kann eine mechanische Schwingung in die Brust eingebracht werden. Damit wird die Ausschüttung des Milchspendereflexhormons Oxytozin unterstützt werden.

Die Erfindung soll nachfolgend anhand von Ausführungsbeispielen näher erläutert werden. In den zugehörigen Zeichnungen zeigt
- Fig. 1: eine Anwendung der Breitfinger-Methode mit einer Hand,
- Fig. 2: eine Anwendung der Breitfinger-Methode mit zwei Händen,
- Fig. 3: eine Anwendung der Spitzfinger-Methode,
- Fig. 4: einen Querschnitt durch einen erfindungsgemäßen Babymundsimulator,
- Fig. 5: eine erste perspektivische Ansicht des Babymundsimulators gemäß Fig. 1,
- Fig. 6: eine zweite perspektivische Ansicht des Babymundsimulators gemäß Fig. 1,
- Fig. 7: eine dritte perspektivische Ansicht des Babymundsimulators gemäß Fig. 1,
- Fig. 7a: einen Babymundsimulator in der Darstellung entsprechend Fig. 7 als mehrteilige Ausgestaltung mit verschiedenen Materialien,
- Fig. 7b: eine Gestaltung der Öffnung in einem Stutzen mit Flansch zum Aufstecken für Flaschen,
- Fig. 7c: eine Gestaltung der Öffnung in einem Stutzen mit Flansch zum Aufstecken für Flaschen mit Entlüftungsöffnung,
- Fig. 7d: eine Gestaltung der Öffnung in einem Stutzen mit Flansch zum Aufschrauben für Flaschen,
- Fig. 8: eine erste perspektivische Ansicht eines Babymundsimulators mit Ausstülpungen,
- Fig. 9: eine zweite perspektivische Ansicht des Babymundsimulators mit Ausstülpungen,
- Fig. 10: ein Ventil in Form eines Rückschlagventils,
- Fig. 11: eine erste perspektivische Ansicht eines Babymundsimulators mit Ausstülpungen und Mamillenschutzkörper,
- Fig. 12: eine zweite perspektivische Ansicht eines Babymundsimulators mit Ausstülpungen und Mamillenschutzkörper,
- Fig. 13: eine Anordnung eines Auffangbehälters,
- Fig. 14: eine Ausgestaltung eines Babymundsimulators für einen Einsatz in einem Adapter gemäß Fig. 15 im Querschnitt,
- Fig. 15: einen Babymundsimulator gemäß Fig. 14, eingesetzt in einen festen Trichter einer konventionellen Milchpumpe,
- Fig. 16: eine Ausgestaltung mit einem Einsteckadapter zum Anschluss des Babymundsimulators an einen Auffangbehälter eines an sich bekannten Pumpsets,
- Fig. 17: eine Ausgestaltung mit dem Einsteckadapter wie in Fig. 16 dargestellt, zum Anschluss an eine konventionelle Milchpumpe, wie in Fig. 15 dargestellt und
- Fig. 18: einen Babymundsimulator für kleine Milchmengen.

Für die Muttermilch-Sekretion sind drei Effekte bekannt, die bewirken, dass Muttermilch aus der Brust kommt:
A) In der Brust selbst wirkt ein Innendruck, der durch die über die Blutbahnen vermittelte Oxytozin-Wirkung verursacht wird. Dabei bewirkt das Oxytozin die sofortige Kontraktion der kleinen Muskeln, die die milchbildenden Zellen (Alveolen) umhüllen. Dieser Effekt wird auch als Milchspendereflex bezeichnet. Er wird am stärksten hervorgerufen und positiv beeinflusst durch Stimulation der Mamille bei gleichzeitiger Entspannung, Wärme, Angst- und Schmerzfreiheit und sanften Massagen an allen geeigneten Punkten des mütterlichen Körpers. Ein wichtiger Unterschied zu den im Folgenden genannten Effekten ist, dass die Milch allein dadurch in Fluss kommt, dass die Milchbläschen innerlich zusammengedrückt werden. Ohne das Oxytozin gäbe es nur sehr wenig Milchsekretion, auch bei stark gefüllter Brust und starkem Saugen.
B) Ein weiterer Effekt, der bewirkt, dass Milch aus der Brust kommt, ist ein deutlich ausgeübter mechanischer Druck auf die weibliche Brust, der nachfolgend als Melkdruck bezeichnet wird, da die Art und Weise Ähnlichkeit mit den Abläufen beim Hand-Melken, wie es aus der Milchtierhaltung bekannt ist, hat. Zum einen (Variante-B1) kann dieser Druck ganz natürlich durch das Kind erzeugt werden, indem es auf die Areola und die Mamille eine melkende Kaubewegung mithilfe von Gaumen, Zunge, Wangen und Kieferleisten ausübt. Zum andern kann ein sehr ähnlicher Druck wie durch das Kind (Variante B1) durch melkende Fingerbewegungen der Mutter (Variante B2) auf die Areola ausgeführt werden. Variante B2 kann beim Stillen zur Variante B1 unterstützend eingesetzt werden. Dieser rhythmische ausgeführte Melkdruck ist im Zusammenhang mit einem wirkenden Milchspendereflex (Effekt A) allein ausreichend, um volle Brüste in kurzer Zeit zu leeren. Bei der Variante-B2 muss die Milch aufgefangen werden, denn die bis zu 20 Milchausführungsgänge verteilen sich über die gesamte Mamillenspitze und die Milch kann in alle Richtungen meterweit spritzen.
C) Der dritte bekannte Effekt, der eine Milchsekretion bewirkt, ist der auf die Brust wirkende Unterdruck oder Saugdruck durch den Säugling. Dieser Effekt wird von herkömmlichen Milchpumpen genutzt. Da die Effekte der B-Varianten nicht oder kaum wirken, wird ein Unterdruck von bis zu 300 mmHg angesetzt, der die Brust schädigen kann. Ein Säugling erzeugt jedoch lediglich einen Unterdruck von 55 bis 150 mmHg.

Diese drei Effekte A, B und C können unterschiedlich zusammen wirken, so dass es zu einer Milchsekretion kommt.

Im Fall, dass ein Säugling direkt an der Brust gestillt wird, wirken alle drei Effekte A, B und C zusammen. Das bedeutet, dass ein gesunder Säugling, wenn Effekt A wirkt, durch sein Saugen Effekt C und gleichzeitig mit Gaumen, Zunge usw. wie oben beschrieben Effekt B1 bewirkt.

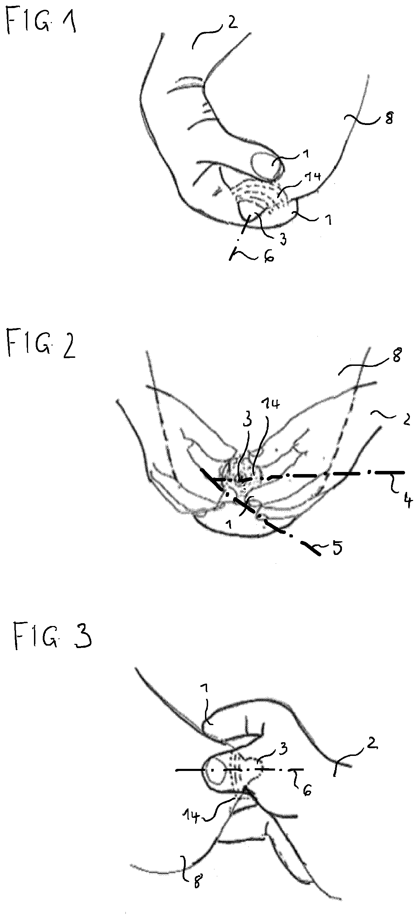

Zur Laktation ohne Säugling sind in Fig. 1 bis Fig. 3 verschiedene Methoden dargestellt. Diese bestehen in einer "Daumen-oben-Finger-unten-Methode", wie in Fig. 1 dargestellt, einer "zwei-bis-zehn-Finger-Methode", wie in Fig. 2 dargestellt und einer "Fünf-Finger-Methode", wie in Fig. 3 dargestellt. Die Methoden nach Fig. 1 und Fig. 2 werden hier als "Breitfinger-Methode" bezeichnet, die Methode nach Fig. 3 als "Spitzfinger-Methode". Mit beiden Methoden wird auf physiologische Art eine rasche und dauerhafte Milchejektion erreicht.

Bei der Breitfingermethode werden die Finger 1 einer Hand 2 radial zur Mamille 3 d.h. im Wesentlichen parallel zu Radiallinie 4 oder tangential, d.h. im Wesentlichen parallel zu einer Tangentiallinie 5 gehalten. Bei der Spitzfingermethode werden die Fingerkuppen 1 der Hand 2 axial zur Mamille 3, d.h. im Wesentlichen parallel zur Achse 6 gehalten, um damit durch eine den Aufnahmeraum 9 umgebenden Wandung 20 hindurch einen Melkdruck auf die Mamille 3 und die angrenzenden Bereiche auszuüben. Der Aufnahmeraum 9 kann bei beiden Methoden von außen mit den Fingern 1 verformt werden, wobei das Aufbringen eines mechanischen Drucks der Stimulation durch die Lippen und Kieferleisten eines Säuglings entspricht. Die innere Dichtmanschette 15 sitzt dabei - den Aufnahmeraum 9 gegenüber der Brust 8 abdichtend - auf der Brust 8 insbesondere im Bereich der Areola 14 auf. Durch die Ausübung des Melkdrucks wird der Aufnahmeraum 9 zusammengedrückt, die Luft entweicht zum Teil aus dem Aufnahmeraum und ein Unterdruck oder ein leichtes Vakuum entsteht. Dies entspricht dem Saugen eines Säuglings. Somit können durch den erfindungsgemäßen Babymundsimulator sämtliche wichtigen Faktoren zur Bewirkung einer Laktation auf natürliche Art und Weise und mit einer eigenen gefühlsmäßigen Rückkopplung simuliert werden.

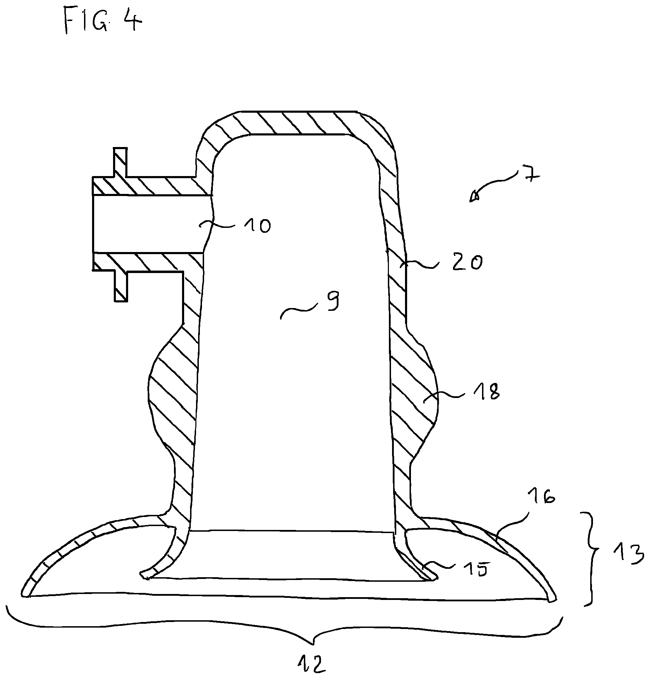

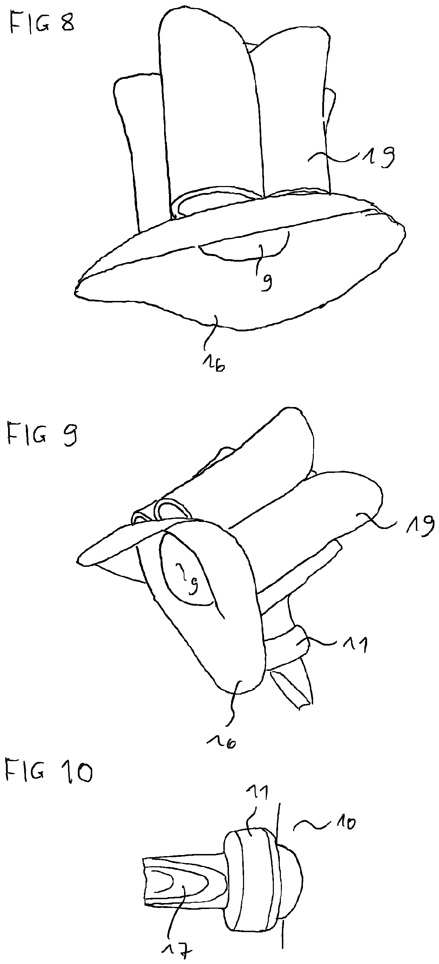

Wie in den Fig. 4 bis Fig. 7 dargestellt, weist der Babymundsimulator 7 für die weibliche Brust 8 einen Aufnahmeraum 9 für die laktierte Milch auf. Der Aufnahmeraum 9 ist mit einer Öffnung 10 versehen, die mit einem Ventil 11 verschlossen ist, welches als Rückschlagventil ausgebildet ist, wie es in Fig. 10 dargestellt ist.

Weiterhin ist der Babymundsimulator 7 mit einer Melkmanschette 12, die einen flexiblen Teil 13 aufweist, versehen, mit dem der Babymundsimulator 1 zumindest auf die Areola 14 der weiblichen Brust 8 dicht aufsetzbar ist. Der flexible Teil 13 ist weiterhin derart ausgebildet, dass ein manueller eine Laktation bewirkender Melkdruck zumindest auf die Mamille 3 der weiblichen Brust 8 oder auch auf die Areola 14 ausführbar ist.

Mittels des flexiblen Teils 13 kann die weibliche Brust 8 mit den Fingern 1 einer Hand 2 so gefasst werden, dass ein manueller, eine Sekretion bewirkender Melkdruck auf die weibliche Brust 8 ausführbar ist, wobei durch die Finger 1 der Mutter im Gegensatz zur konventionellen Pumpe ein rhythmischer, genauestens dosierbarer Melkdruck auf die Areola 14 oder den angrenzenden Brustbereich ausgeübt werden kann und sowohl eine Stimulation der Nerven als auch effektive Leerungsförderung bewirkt. Damit wird auf physiologische Art eine rasche und dauerhafte Milchejektion erreicht. Bei Schwellungen der Brust 8, insbesondere beim Einschießen der Milch oder bei Milchstau, kann dabei auch leicht ein minutenlanger sanfter Druck auf die Areola 14 ausgeübt werden, um die stauende Lymphflüssigkeit wegzubewegen. Der ausgeübte Melkdruck bewirkt weiterhin eine deutliche Erhöhung der Menge an Milch, die in gleicher Zeit gewonnen werden kann. Das führt - neben der Zeiteinsparung - bei ausreichend häufiger Anwendung dazu, dass das nahezu volle Potential der Mutter zur Milchbildung ausschöpfbar ist.

Wie in den Zeichnungen dargestellt, ist die Melkmanschette 12 als Doppelmanschette ausgebildet, wobei eine den Aufnahmeraum 9 beim bestimmungsgemäßen Einsatz gegenüber der Areola 14 abdichtenden inneren Dichtmanschette 15 und eine beim bestimmungsgemäßen Einsatz den Babymundsimulator 7 auf der Brust 8 mechanisch fixierende Fixierungsmanschette 16 vorgesehen ist. Die Fixierungsmanschette 16 ist größer als die Dichtmanschette 15 ausgebildet und umgibt die Dichtmanschette (15).

Mittels des Rückschlagventils 11 oder einer Verschlusskappe ist der Aufnahmeraum 9 als Vakuumraum ausgebildet.

Der Aufnahmeraum 9 kann bei beiden Methoden von außen mit den Fingern 1 verformt werden. Die innere Dichtmanschette 15 sitzt dabei auf der Brust 8 insbesondere im Bereich der Areola 14 auf. Die Verformung bewirkt ein Zusammendrücken des Aufnahmeraums 9. Dadurch entweicht die Luft zum Teil aus dem Aufnahmeraum 9 und ein Unterdruck oder ein leichtes Vakuum entsteht.

Die äußere Fixierungsmanschette 16 braucht keine Dichtwirkung zu entfalten. Sie dient vielmehr der mechanischen Fixierung, indem die Hand 2 oder die Finger 1 die Fixierungsmanschette 16 auf die Brust 8 zu drückt. Der Babymundsimulator 7 wird damit fixiert.

Das Ventil 11 ist als Rückschlagventil ausgebildet. Wie in Fig. 10 dargestellt, weist dieses Ventil Dichtlippen 17 auf, die beim Zusammendrücken des Aufnahmeraums 9 durch die entweichende Luft auseinandergedrückt werden und damit den Weg für die austretende Luft freigeben. Entlasten die Finger 1 den Aufnahmeraum 9 wieder, hat dieser infolge des flexiblen Materials das Bestreben, in seine alte Form zurückzukehren, wodurch das Vakuum entsteht. Ist dann das Vakuum in dem Aufnahmeraum 9 entstanden, drückt der äußere Luftdruck die Dichtlippen 17 zusammen und die Öffnung 10 des Aufnahmeraumes 9 wird verschlossen.

Zum mechanischen Schutz der Mamille 3 ist ein den Aufnahmeraum 9 umschließender Verstärkungsring 18 vorgesehen.

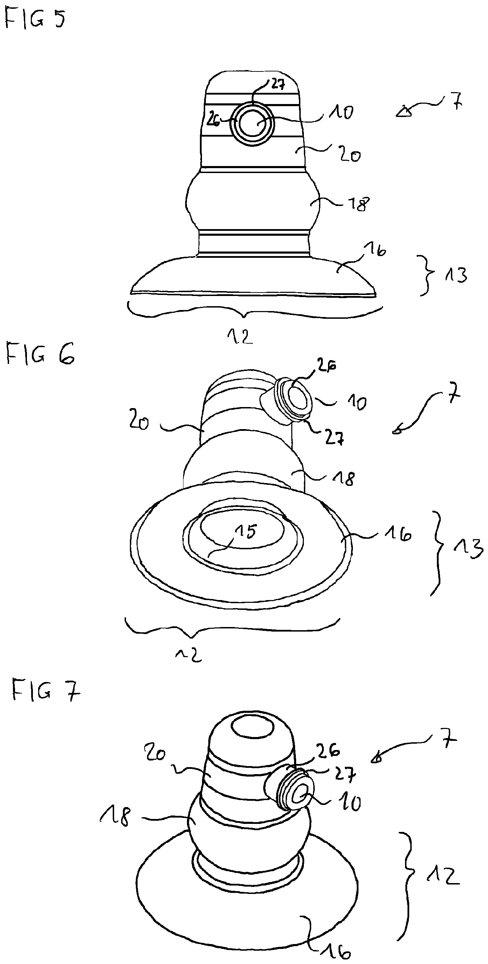

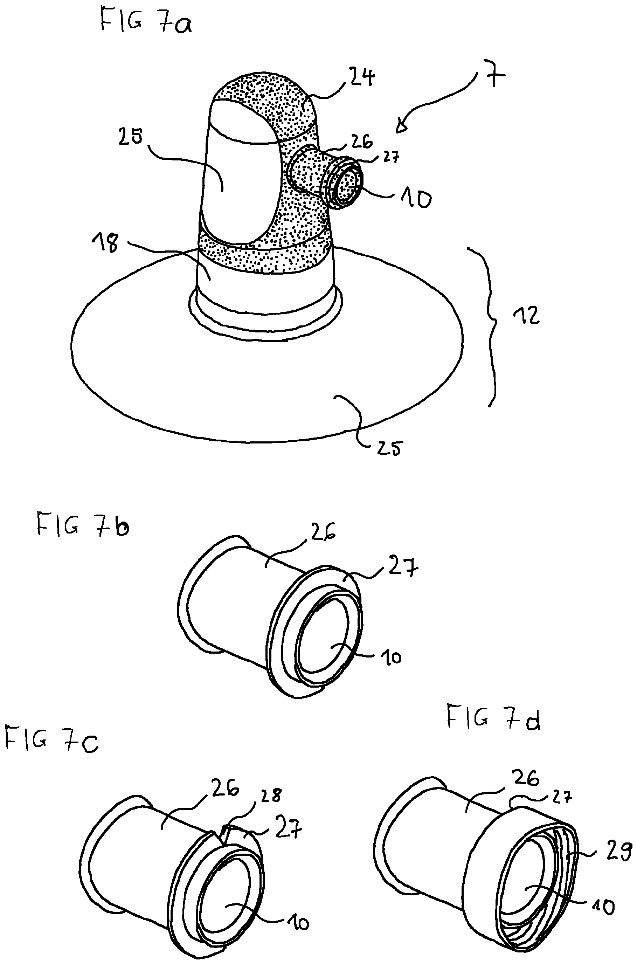

Fig. 7a zeigt eine Gestaltung, bei der der Babymundsimulator 7 aus zumindest zwei verschiedenen Materialien besteht. So ist ein erster Bereich 24, der in der Zeichnung punktiert dargestellt ist, aus einem im Verhältnis zu einem zweiten Bereich 25 steiferen Material gefertigt.

Fig. 7b zeigt eine Gestaltung der Öffnung in einem Stutzen 26. Der Stutzen 26 ist mit einem Flansch 27 zum Aufstecken eines nicht näher dargestellten Beutels versehen. So kann ein Rand des Beutels über den Flansch gezogen werden und befestigt diesen am Stutzen 26 und dichtet ihn gleichzeitig ab.

Wie in Fig. 7c dargestellt, ist der Flansch 27 an seinem Rand mit einer Einkerbung 28 versehen. Diese bildet bei einem aufgezogenen Beutel mit dem Rand des Beutels eine Öffnung, durch die Luft aus dem Beutel entweichen kann, um somit einen Druckausgleich beim Einströmen der Milch zu schaffen.

Wie in Fig. 7d dargestellt, ist der Flansch mit einem Gewindeansatz 29 versehen, der ein Innengewinde aufweist, in das ein Außengewinde einer nicht näher dargestellten Flasche einschraubbar ist.

Der Babymundsimulator 7, wie er in den Figuren 8, 9, 11 und 12 dargestellt ist, ist dadurch ausgestaltet, dass der Aufnahmeraum 9 an seiner Außenseite mit Ausstülpungen 19 versehen ist, in denen zumindest Teile der Finger 1 einer Hand 2 aufnehmbar sind. Mit diesen Ausstülpungen 19 lässt sich die weibliche Brust 8 oder die Areola 14 mit den Fingern 1 der Hand 2 durch die Wandung 20 des Aufnahmeraumes 9 hindurch leicht umschließen und ein manueller, eine Laktation bewirkender Melkdruck kann auf die Mamille und die angrenzenden Bereiche mit den Fingern ausgeführt werden.

In die Ausstülpungen 19 sind zumindest die Fingerkuppen 1 der Hand 2 aufnehmbar, wobei die Ausstülpungen 19 den Aufnahmeraum 9 umgeben.

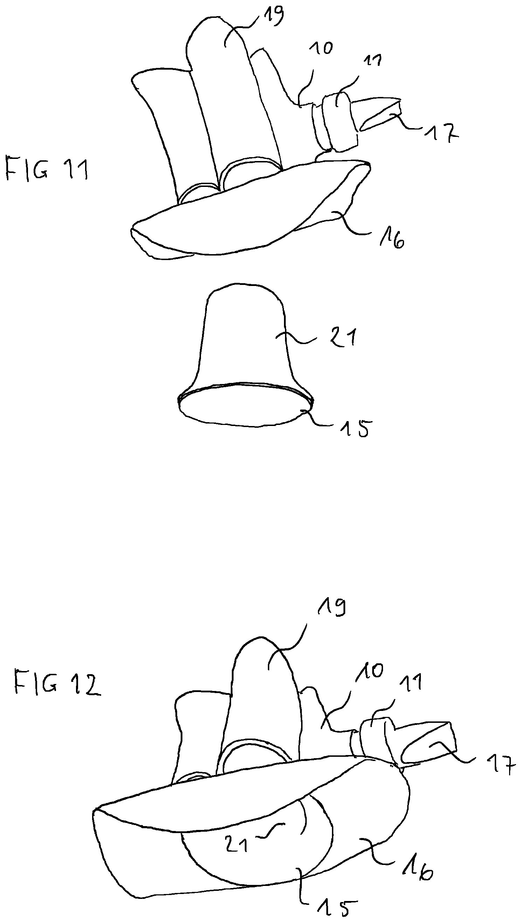

In dem Ausführungsbeispiel, welches in Fig. 11 und 12 dargestellt ist, ist ein in den Aufnahmeraum 9 einsteckbarer Mamillenschutzkörper 21 aus einem im Vergleich zur Melkmanschette 12 oder zur Wandung 20 des Aufnahmeraums 9 steiferem Material vorgesehen. Dieser Mamillenschutzkörper 21 bietet - ähnlich dem bereits oben beschriebenen Verstärkungsring 18 - einen Schutz der Mamille 3 vor einer zu kräftigen Melkbewegung. Der Mamillenschutzkörper 21 ist bei einem Babymundsimulator 7 einsetzbar, der für die Spitzfingermethode mit äußeren Ausstülpungen 19 versehen ist, da bei dieser Lösung das Anbringen eines festen Verstärkungsringes 18 nicht sinnvoll ist.

Der Mamillenschutzkörper 21 ist in den Aufnahmeraum 9 dicht einzustecken, wobei die Dichtmanschette 15 mit dem Mamillenschutzkörper 21 verbunden ist.

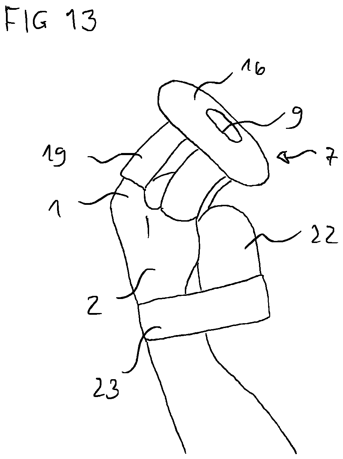

Wie in Fig. 13 dargestellt, kann mit dem Aufnahmeraum 9 ein Auffanggefäß 22 verbunden sein. In dieses Auffanggefäß 22 kann dann die laktierte Milch abfließen. Dabei ist das Auffanggefäß 22 mit einer Armmanschette 23 zur Befestigung insbesondere am Handgelenk versehen. Die Verbindung des Auffanggefäßes 22 mit dem Aufnahmeraum 9 kann durch eine Schraub- oder eine Steckverbindung realisiert werden.

Für kleine Milchmengen kann es auch möglich sein, den Aufnahmeraum 9 mit einem Verschluss zu verschließen. In diesem Falle kann auch das Ventil 11 entfallen.

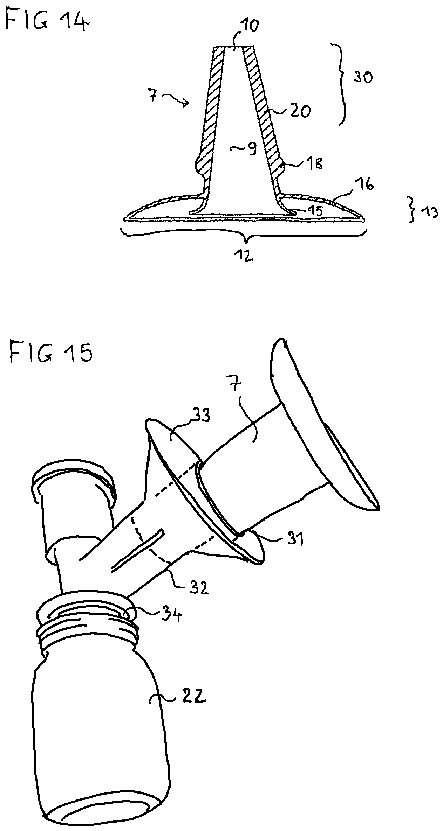

Fig. 14 zeigt eine andere Ausführungsform einer Verbindung des Aufnahmeraumes 9 mit dem Auffanggefäß 22. Der Aufbau des Babymundsimulators entspricht grundsätzlich dem Aufbau gemäß Fig. 5. Hier ist jedoch die Öffnung 10 auf der der Melkmanschette abgewandten Seite der Wandung 20 angeordnet und die Außenseite der Wandung 20 zumindest in einem an die Öffnung 10 angerechneten Bereich 30 konisch gestaltet.

Wie in Fig. 15 dargestellt, dient dieser Bereich 30 dem Einstecken in eine Aufnahmeöffnung 31 eines festen Trichters 32, 33 und 34 einer konventionellen Milchpumpe.

Bei dieser Ausführungsform wird der Unterdruck durch die elektrische Milchpumpe (nicht dargestellt) hergestellt. Bei einer Milchpumpe ohne den Einsatz der Erfindung wird üblicherweise der Kragen 33 auf den Bereich der Mamille 3 aufgesetzt. Mit Hilfe der Erfindung wird nun der erfindungsgemäße Babymundsimulator 7 "zwischengeschaltet". Die Mutter kann mit Hilfe des Adapters 32 während des Abpumpvorganges die Brust durch den Babymundsimulator 7 stimulieren, massieren und gleichzeitig melkende Fingerbewegungen ausführen. Die innere Manschette 15 dient zur Abdichtung des elektrisch hergestellten Unterdruckes gegenüber der Brust. Die äußere Manschette 16 dient der Fixierung des Adapters 32 an der Brust und ermöglicht die Melkbewegungen der Finger. Der Mamillenschutzring 18 sorgt für das Offenhalten des Mamillenbereiches und damit sowohl für Schmerzfreiheit als auch Weitung der Mamille zur Verstärkung der Milchejektion.

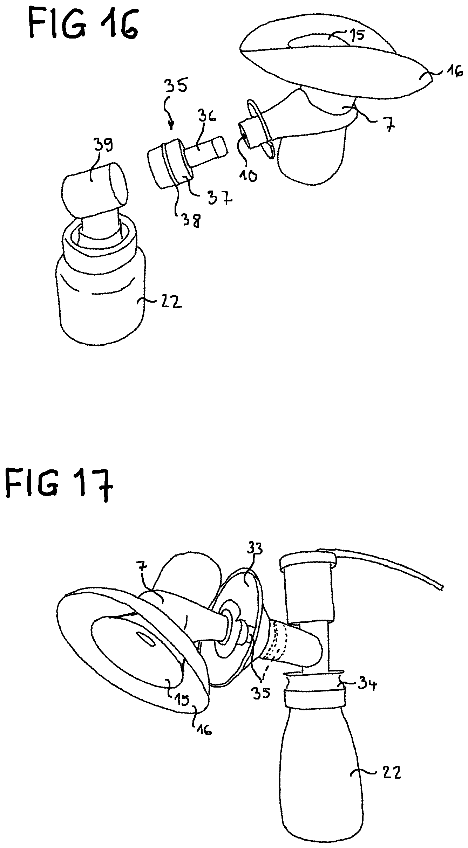

Fig. 16 zeigt die Realisierung eines Einsteckadapters 35. Dieser weist ein Einsteckrohr 36 auf, das in die Öffnung 10 des Babymundsimulators einsteckbar ist. Das Einsteckrohr 36 ist mit einer durchgehenden Öffnung mit einem Einsteckstück 37 versehen, welches auf seiner Außenseite mit einer Ringdichtung 38 versehen. Durch eine entsprechende Gestaltung des Einsteckstücks 37 ist es möglich, den Babymundsimulator an Auffangbehälter 22 anderer Hersteller anzuschließen und somit einen universellen Einsatz zu ermöglichen. In der hier dargestellten Form ist das Einsatzstück so ausgebildet, dass es in den Schlauchanschluss 39 eines Pumpsets eines anderen Herstellers einsteckbar ist.

Fig. 17 zeigt einen an einer Milchpumpe, wie sie in Fig. 15 bereits dargestellt wurde, montierten Babymundsimulator 7 und Einsatz eines Einsteckadapters 35. Hierbei wurde das Einsteckstück so konzipiert, dass es zu der Öffnung des Kragens 33 kompatibel ist und dort eingesteckt werden kann.

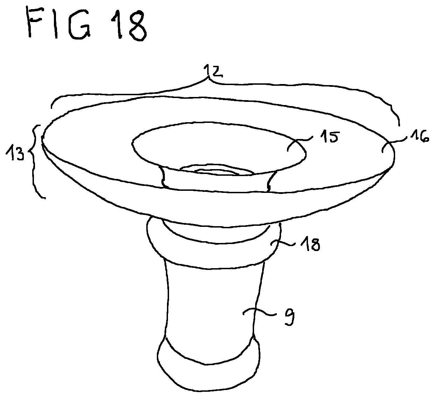

Fig. 18 zeigt einen Babymundsimulator 7 für kleinere Milchmengen. Hier zeigt der Aufnahmeraum 9 keine Öffnung 10. Dieser Babymundsimulator wird mit der Dichtmanschette 15 auf die Brust aufgesetzt, leicht zusammengedrückt, wodurch ein Unterdruck entsteht, und durch die Fixierungsmanschette 16 festgehalten. Die durch die Melkbewegung über die Melkmanschette 12 und den Unterdruck austretende Milch wird einfach in dem Aufnahmeraum 9 aufgefangen. Ist dieser gefüllt oder erfolgt keine Laktation mehr, wird der Babymundsimulator 7 abgenommen und der Aufnahmeraum 9 entleert.

### Bezugszeichenliste

- 1: Fingerkuppe, Finger
- 2: Hand
- 3: Mamille
- 4: Radiallinie
- 5: Tangentiallinie
- 6: Achse
- 7: Babymundsimulator
- 8: Brust
- 9: Aufnahmeraum
- 10: Öffnung
- 11: Ventil
- 12: Melkmanschette
- 13: flexibler Teil
- 14: Areola
- 15: innere Dichtmanschette
- 16: Fixierungsmanschette
- 17: Dichtlippe
- 18: Verstärkungsring
- 19: Ausstülpung
- 20: Wandung
- 21: Mamillenschutzkörper
- 22: Auffanggefäß
- 23: Armmanschette
- 24: erster Bereich
- 25: zweiter Bereich
- 26: Stutzen
- 27: Flansch
- 28: Einkerbung
- 29: Gewindeansatz
- 30: Bereich
- 31: Aufnahmeöffnung
- 32: Adapter
- 33: Kragen
- 34: Gewindeansatz
- 35: Einsteckadapter
- 36: Einsteckrohr
- 37: Einsteckstück
- 38: Ringdichtung
- 39: Schlauchanschluss

## Patentansprüche

1. Babymundsimulator für die weibliche Brust mit einem Aufnahmeraum für die laktierte Milch und mit einer Melkmanschette, die einen flexiblen Teil aufweist, mit dem der Babymundsimulator zumindest auf die Mamillen der weiblichen Brust dicht aufsetzbar ist und die derart ausgebildet ist, dass ein manueller eine Laktation bewirkender Melkdruck auf die Mamille und die angrenzenden Bereiche ausführbar ist, **dadurch gekennzeichnet, dass** die Melkmanschette (12) als Doppelmanschette ausgebildet ist, wobei eine den Aufnahmeraum (9) beim bestimmungsgemäßen Einsatz gegenüber der Areola (14) abdichtenden inneren Dichtmanschette (15) und eine beim bestimmungsgemäßen Einsatz den Babymundsimulator (7) auf der Brust (8) mechanisch fixierende, keine Dichtfunktion aufweisende Fixierungsmanschette (16) vorgesehen ist und dass der Aufnahmeraum (9) als Vakuumraum ausgebildet ist.

2. Babymundsimulator nach Anspruch 1, **dadurch gekennzeichnet, dass** die Fixierungsmanschette (16) größer als die Dichtmanschette (15) ist und die Dichtmanschette (15) umgibt.

3. Babymundsimulator nach Anspruch 1 oder 2, **dadurch gekennzeichnet, dass** der Aufnahmeraum (9) eine Öffnung aufweist, die mit einem Ventil (11), insbesondere einem Rückschlagventil, welches insbesondere als Lippenventil (17) ausgebildet ist, versehen ist.

4. Babymundsimulator nach einem der Ansprüche 1 bis 3, **dadurch gekennzeichnet, dass** ein den Aufnahmeraum (9) umschließender Verstärkungsring (18) vorgesehen ist.

5. Babymundsimulator nach einem der vorstehenden Ansprüche, **dadurch gekennzeichnet, dass** der Babymundsimulator (7) zwei oder mehrteilig ausgebildet ist.

6. Babymundsimulator nach Anspruch 1, **dadurch gekennzeichnet, dass** der flexible Teil (13) aus einem weichelastischen Material ausgebildet ist.

7. Babymundsimulator nach Anspruch 5 oder 6, **dadurch gekennzeichnet, dass** die Teile des Babymundsimulators (7) aus verschiedenen Materialen bestehen, die miteinander verbunden sind.

8. Babymundsimulator nach einem der Ansprüche 5 bis 7, **dadurch gekennzeichnet, dass** ein in den Aufnahmeraum (9) einsteckbarer Mamillenschutzkörper (21) vorgesehen ist.

9. Babymundsimulator nach Anspruch 8, **dadurch gekennzeichnet, dass** der Mamillenschutzkörper (21) in den als Vakuumraum ausgebildeten Aufnahmeraum (9) dicht einbringbar ist und die Dichtmanschette (15) mit dem Mamillenschutzkörper (21) verbunden ist.

10. Babymundsimulator nach einem der vorherigen Ansprüche, **dadurch gekennzeichnet, dass** der Aufnahmeraum (9) an seiner Außenseite mit Ausstülpungen (19) versehen ist, in denen zumindest Teile der Finger (1) aufnehmbar sind.

11. Babymundsimulator nach Anspruch 10, **dadurch gekennzeichnet, dass** im Verhältnis zu den Ausstülpungen (19) steifere Offenhalter vorgesehen sind, die in die Ausstülpungen anstelle der Fingerkuppen (1) einsteckbar sind.

12. Babymundsimulator nach einem der vorstehenden Ansprüche, **dadurch gekennzeichnet, dass** mit dem Aufnahmeraum (9) ein Auffanggefäß (22) verbindbar ist.

13. Babymundsimulator nach Anspruch 12, **dadurch gekennzeichnet, dass** das Ventil (11) oder ein zweites Ventil zwischen dem Aufnahmeraum (9) und dem Auffanggefäß (22) angeordnet ist.

14. Babymundsimulator nach Anspruch 13, **dadurch gekennzeichnet, dass** das Auffanggefäß (22) mit einer Armmanschette (23) zur Befestigung insbesondere am Handgelenk oder am Unterarm versehen ist.

15. Babymundsimulator nach einem der vorhergehenden Ansprüche, **dadurch gekennzeichnet, dass** eine einen mechanischen Vibrator aufnehmbare Aufnahmetasche vorgesehen ist.

## Claims

1. A baby's mouth simulator for the female breast, having a receptacle for the lactated milk and having a milking cuff which has a flexible part, with which the baby's mouth simulator can be positioned and sealed onto at least the nipples of the female breast and which is configured in a manner such that a manual milking pressure which brings about lactation can be exerted on the nipple and the adjoining regions, **characterized in that** the milking cuff (12) is configured as a double cuff, wherein an inner sealing cuff (15) which seals the receptacle (9) against the areola (14) when used appropriately and a fixation cuff (16) which mechanically fixes the baby's mouth simulator (7) on the breast (8) without having a sealing function when used appropriately are provided, and **in that** the receptacle (9) is configured as a vacuum chamber.

2. The baby's mouth simulator as claimed in claim 1, **characterized in that** the fixation cuff (16) is larger than the sealing cuff (15) and surrounds the sealing cuff (15).

3. The baby's mouth simulator as claimed in claim 1 or claim 2, **characterized in that** the receptacle (9) has an opening which is provided with a valve (11), in particular a one-way valve, which in particular is configured as a lip valve (17) .

4. The baby's mouth simulator as claimed in one of claims 1 to 3, **characterized in that** a reinforcing ring (18) is provided which surrounds the receptacle (9).

5. The baby's mouth simulator as claimed in one of the preceding claims, **characterized in that** the baby's mouth simulator (7) is constructed in two or more parts.

6. The baby's mouth simulator as claimed in claim 1, **characterized in that** the flexible part (13) is constructed from a soft resilient material.

7. The baby's mouth simulator as claimed in claim 5 or claim 6, **characterized in that** the parts of the baby's mouth simulator (7) consists of different materials which are connected together.

8. The baby's mouth simulator as claimed in one of claims 5 to 7, **characterized in that** a nipple protecting member (21) is provided which can be inserted into the receptacle (9) .

9. The baby's mouth simulator as claimed in claim 8, **characterized in that** the nipple protecting member (21) can be inserted into the receptacle (9) configured as a vacuum chamber in a sealed manner and the sealing cuff (15) is connected to the nipple protecting member (21).

10. The baby's mouth simulator as claimed in one of the preceding claims, **characterized in that** the outside of the receptacle (9) is provided with protrusions (19) in which at least parts of the fingers (1) can be accommodated.

11. The baby's mouth simulator as claimed in claim 10, **characterized in that** openers are provided which are stiffer than the protrusions (19) and which can be inserted into the protrusions instead of the fingertips (1).

12. The baby's mouth simulator as claimed in one of the preceding claims, **characterized in that** a collecting container (22) can be connected to the receptacle (9).

13. The baby's mouth simulator as claimed in claim 12, **characterized in that** the valve (11) or a second valve is disposed between the receptacle (9) and the collecting container (22) .

14. The baby's mouth simulator as claimed in claim 13, **characterized in that** the collecting container (22) is provided with an arm cuff (23) in particular for fastening to the wrist or the forearm.

15. The baby's mouth simulator as claimed in one of the preceding claims, **characterized in that** a collecting bag having a mechanical vibrator is provided.

## Revendications

1. Simulateur de bouche de nourrisson pour la poitrine féminine avec un espace de logement pour le lait issu de la lactation et avec un manchon de traite, qui comprend une partie flexible, avec laquelle le simulateur de bouche de nourrisson peut être posé de manière étanche au moins sur les mamelons de la poitrine féminine et qui est conçue de façon à ce qu'une pression de traite manuelle provoquant une lactation puisse être appliquée sur le mamelon et les parties voisines, **caractérisé en ce que** le manchon de traite (12) est conçu comme un manchon double, dans lequel un manchon étanche interne (15) étanchéifiant l'espace de logement (9), dans le cas d'une utilisation conforme à l'usage prévu, par rapport à l'aréole (14) et un manchon de fixation (16), ne présentant aucune fonction d'étanchéité et fixant mécaniquement, dans le cas d'une utilisation conforme à l'usage prévu, le simulateur de bouche de nourrisson (7) sur la poitrine (8), sont prévus et **en ce que** l'espace de logement (9) est conçu comme un espace à vide.

2. Simulateur de bouche de nourrisson selon la revendication 1, **caractérisé en ce que** le manchon de fixation (16) est plus grand que le manchon d'étanchéité (15) et entoure le manchon d'étanchéité (15).

3. Simulateur de bouche de nourrisson selon la revendication 1 ou 2, **caractérisé en ce que** l'espace de logement (9) comprend une ouverture qui est munie d'une soupape (11), plus particulièrement d'un clapet anti-retour, qui est conçue plus particulièrement comme une soupape à lèvres (17).

4. Simulateur de bouche de nourrisson selon l'une des revendications 1 à 3, **caractérisé en ce qu'**un anneau de renforcement (18) entourant l'espace de logement (9) est prévu.

5. Simulateur de bouche de nourrisson selon l'une des revendications précédentes, le simulateur de bouche de nourrisson (7) étant **caractérisé en ce qu'**il est réalisé en deux parties ou plus.

6. Simulateur de bouche de nourrisson selon la revendication 1, **caractérisé en ce que** la partie flexible (13) est constituée d'un matériau élastique souple.

7. Simulateur de bouche de nourrisson selon la revendication 5 ou 6, **caractérisé en ce que** les parties du simulateur de bouche de nourrisson (7) sont constituées de différents matériaux qui sont reliés entre eux.

8. Simulateur de bouche de nourrisson selon l'une des revendications 5 à 7, **caractérisé en ce qu'**un élément de protection de mamelon (21) insérable dans l'espace de logement (9) est prévu.

9. Simulateur de bouche de nourrisson selon la revendication 8, **caractérisé en ce que** l'élément de protection de mamelon (21) peut être inséré de manière étanche dans l'espace de logement (9) conçu comme un espace à vide et le manchon d'étanchéité (15) est relié avec l'élément de protection de mamelon (21).

10. Simulateur de bouche de nourrisson selon l'une des revendications précédentes, **caractérisé en ce que** l'espace de logement (9) est muni, sur son côté externe, de protubérances (19) dans lesquelles au moins des parties des doigts (1) peuvent être logées.

11. Simulateur de bouche de nourrisson selon la revendication 10, **caractérisé en ce que** des dispositifs de maintien d'ouverture, plus rigides que les protubérances (19), sont prévus, qui peuvent être insérés dans les protubérances à la place des bouts des doigts (1).

12. Simulateur de bouche de nourrisson selon l'une des revendications précédentes, **caractérisé en ce qu'**un récipient de collecte (22) peut être relié avec l'espace de logement (9).

13. Simulateur de bouche de nourrisson selon la revendication 12, **caractérisé en ce que** la soupape (11) ou une deuxième soupape est disposée entre l'espace de logement (9) et le récipient de collecte (22).

14. Simulateur de bouche de nourrisson selon la revendication 13, **caractérisé en ce que** le récipient de collecte (22) est muni d'un manchon de bras (23) pour une fixation plus particulièrement au poignet ou à l'avant-bras.

15. Simulateur de bouche de nourrisson selon l'une des revendications précédentes, **caractérisé en ce qu'**une poche de logement logeant un vibrateur mécanique est prévue.
